# EUROPEAN PATENT SPECIFICATION

(11) **EP 1 446 126 B1**
(45) Date of publication and mention of the grant of the patent: **07.07.2010**
(21) Application number: 02777359.7
(22) Date of filing: 21.11.2002
(51) Int. Cl.: A61K 31/546, A61K 9/00, A61K 9/16

(54) **COATED PARTICULATE CEFUROXIME AXETIL COMPOSITIONS**
BESCHICHTETE TEILCHENFÖRMIGE CEFUROXIMEAXETIL-ZUSAMMENSETZUNGEN
COMPOSITIONS DE CEFUROXIME AXETIL PARTICULAIRE ENROBE

(30) Priority: 23.11.2001 EP 01500277
(43) Date of publication of application: 18.08.2004
(73) Proprietor: GLAXO GROUP LIMITED, Greenford, Middlesex UB6 0NN (GB)
(72) Inventor: FERNANDEZ, Matilde, Ibanez, 28760 Tres Cantos (ES); GARRIZ, Emilio, Sanz, 28760 Tres Cantos (ES)
(74) Representative: Connell, Anthony Christopher
(86) International application number: PCT/EP2002/013150
(87) International publication number: WO 2003/043638

(56) References cited:
- WO-A-00/30647
- WO-A-99/32124
- GB-A- 1 323 161
- GB-A- 1 571 683
- GB-A- 2 127 401
- US-A- 4 865 851
- US-A- 5 494 681

## Description

### Field of the Invention

This invention is concerned with compositions, in particular pharmaceutical compositions containing the 1-acetoxyethyl ester of cefuroxime, which has the approved name 'cefuroxime axetil'.

### Background to the Invention

Cefuroxime, as disclosed in British Patent Specification No. 1453049, is a valuable broad spectrum antibiotic characterised by high activity against a wide range of gram-positive and gram-negative micro-organisms, this property being enhanced by the very high stability of the compound to β-lactamases produced by a range of gram negative micro-organisms. Cefuroxime and its salts are principally of value as injectable antibiotics since they are poorly absorbed from the gastro-intestinal tract.

Esterification of the carboxyl group of cefuroxime as a 1-acetoxyethyl ester to give cefuroxime axetil improves the effectiveness on oral administration as disclosed in British Patent Specification No. 1571683. The presence of the 1-acetoxyethyl esterifying group results in significant absorption of the compound from the gastro-intestinal tract, whereupon the esterifying group is hydrolysed by enzymes present in, for example, serum and body tissues to yield the antibiotically active acid. It is particularly advantageous to employ cefuroxime axetil in an amorphous form as disclosed in British Patent Specification No. 2127401.

A convenient means of presenting antibiotics for oral administration is in the form of granules which may be administered as a solution or suspension or taken with a draught of water. Solutions or suspensions of granules as, for example, a syrup are particularly convenient for oral administration of antibiotics to children. However, cefuroxime axetil has an extremely bitter taste which is long lasting and which cannot be adequately masked by the addition of sweeteners and flavours to conventional granule presentations.

Another problem arises from the tendency of cefuroxime axetil, both in crystalline form and the amorphous form to form a gelatinous mass when contacted with aqueous media. This gelling effect is temperature dependent but does occur at temperatures of about 37°C, i.e. at the physiological temperatures at which the disintegration of an orally administered granule would take place. Where there is a relatively slow dispersion of cefuroxime axetil into the surrounding aqueous medium following ingestion there is still the risk that the cefuroxime axetil present in the composition may gel. Such gel formation would lead to the poor dissolution of the cefuroxime axetil and hence poor absorption from the gastrointestinal tract - ie - low bioavailability. In the case of granule formulations the use of particles of small diameter and high surface area is desirable to avoid such gelling.

In the formulation of cefuroxime axetil into granules it is important to avoid the release of the drug into any liquid medium in which it is suspended or indeed into the mouth when administering. Such problems may be minimised by formulating the cefuroxime axetil as lipid coated particles.

GB 2204792 discloses a particulate formulation in which the above problems are addressed. This patent discloses a composition comprising cefuroxime axetil in particulate form, the particles being provided with integral coatings of a lipid or a mixture of lipids which are insoluble in water and which serve to mask the bitter taste of cefuroxime axetil upon oral administration but which disperse or dissolve on contact with gastrointestinal fluid. The formulated coated particles break down upon contact with gastrointestinal fluid, thus allowing rapid dispersion and dissolution in the gastrointestinal tract.

WO 94/25006 discloses a method of masking the flavour of bitter tasting drugs in particulate form by mixing the drug with a lipid at a temperature below that where significant drug degradation occurs. To the drug and lipid mixture is added an emulsifier and surfactant, a polymer solution, and a dilution solution to form the stable taste-masked drug composition.

WO 00/076479 discloses taste masked compositions comprising a bitter tasting active, such as cefuroxime axetil, and two enteric polymers, namely methacrylic acid copolymer and phthalate polymer which are dissolved in a solvent system and subsequently dried to form a "solid solution" matrix in which the drug is kept in a finely dispersed state within the polymers, preventing the exposure of the bitter tasting drug to the taste buds.

The applicants currently market an oral suspension composition comprising cefuroxime axetil, the particles being provided with integral coatings of a lipid in the UK under the tradename Zinnat ^{™} and in the US under the tradename Ceftin ^{™}. This oral suspension composition comprises, in addition to cefuroxime axetil, the inactive ingredients stearic acid, tutti frutti flavour, a binding agent (Povidone K30) and sucrose as a bulk sweetener.

Although the lipid coating goes some way to mask the bitter taste of the cefuroxime axetil upon oral administration, cefuroxime axetil is so bitter that these suspensions and compositions still have a bitter taste and prove a particular problem for administration to children. In addition, the suspensions may have a "gritty" feeling in the mouth making them less palatable than other antibiotic suspensions. Both of these factors may affect patient compliance because, particularly in children, less palatable antibiotics are likely to be discontinued as soon as the patient is well rather than continuing the course for the prescribed duration. In view of the above, there is a need for improved cefuroxime axetil suspensions to reduce the significant bitter taste and to improve mouth "feel".

The present inventors have surprisingly now found a way to further improve the taste of the cefuroxime axetil used to form a suspension such that its unfavourable bitter taste may prove more acceptable. Advantageously, the overall "feel" in the mouth of the cefuroxime axetil suspension formulation is also improved in terms of less grittiness and is more easy to swallow.

### Summary of the Invention

Accordingly the present invention provides a composition comprising cefuroxime axetil in particulate form, the particles being provided with integral coatings of lipid or mixture of lipids which are insoluble in water and which disperse or dissolve on contact with gastrointestinal fluid **characterised in that** the composition further comprises a sweetener system and a texture modifier in amounts sufficient to mask the bitter taste of cefuroxime axetil.

More particularly, the present invention provides a composition comprising cefuroxime axetil in particulate form, the particles being provided with integral coatings of lipid or mixture of lipids which are insoluble in water and which disperse or dissolve on contact with gastrointestinal fluid, a bulk sweetener and a binding agent, **characterised in that** the composition further comprises in amounts sufficient to mask the bitter taste of cefuroxime axetil
- acesulfame potassium and aspartame, present in an amount together of 0.1 to 10% by weight of the final granule composition, and
- a texture modifier which is xanthan gum, present in an amount together of 0.01 to 5% by weight of the final granule composition.

Advantageously, it has been found that the sweetener system and texture modifier act synergistically to overcome both the bitter taste and also improve mouth "feel" thereby aiding patient compliance. As indicated above, the sweetener system overcomes the bitter taste by producing an initial sweet taste in the mouth. However, the simultaneous use of the texture modifier helps to provide a creamier texture improving mouth "feel" and, in addition, reducing the number of lipid coated particles left in the mouth when the preparation is swallowed further reducing the bitter taste effect. Using individual sweeteners or the texture modifier alone, would not produce such a significant improvement in both taste masking and mouth "feel". Applicants have discovered that these beneficial effects are only produced when the sweeteners are combined and are further improved when the texture modifier is used in a synergistic combination.

### Detailed description of the Invention

Suitable lipid or mixtures of lipid coating for the cefuroxime axetil particles together with methods for preparing lipid coated particles of cefuroxime axetil are described for example in GB 2204792. A particularly preferred lipid coating is stearic acid in admixture with palmitic acid in a ratio in the range 3:7 to 7:3 by weight, more preferably 1:1 by weight.

The composition of the invention may contain cefuroxime axetil in crystalline form, in a mixture of crystalline and amorphous forms and more preferably in the amorphous form, for example as described in GB 2127401.

As described in GB2204792 the cefuroxime axetil particles may be undercoated with a substance with coating properties in order to protect the cefuroxime axetil where it may be chemically sensitive to the lipid with which it is coated. As described in GB2204792 undercoated particles in which the cefuroxime axetil is present at a concentration of 10-30%, for example about 20%, may conveniently be used for coating by the lipid.

Suitable methods of coating the cefuroxime axetil particles with the lipid or mixture of lipids are disclosed in GB 2204792. The patent also discloses the preferred sizes of the lipid coated particles. When the cefuroxime axetil for dispersion in the lipid is undercoated the lipid coating preferably represents 20-80% by weight, more preferably 35-65% by weight of the coated particles.

The lipid coated particles according to the invention will preferably contain from 5 to 90%, more preferably from 5 to 50% and still more preferably from 5 or 10 to 30% by weight of cefuroxime axetil. Where the cefuroxime axetil is first undercoated the lipid coated particles most preferably contain from 5 to 15% by weight of cefuroxime axetil; where no undercoating is employed the lipid coated particles most preferably contain from 10 to 30% by weight of cefuroxime axetil.

By "sweetener system" is a mixture of acesulfame potassium and aspartame.

The sweetener system comprises between about 0.1-10% by weight of the final granule composition, more preferably about 0.3 to 5% by weight. The ratios between the two sweeteners are in the range of about 1:10 to 10:1 by weight.

The mixture of acesulfame potassium and aspartame is preferably in a weight ratio of about 1:1.

The composition additionally comprises a "texture modifier" comprising one or more thickening agents. The texture modifier is added in addition to any thickeners or binding agents which may optionally form part of the composition and therefore constitutes an essential feature of the invention. By "texture modifier"is meant a thickening agent, or combination of thickening agents, which helps to improve the texture of the cefuroxime axetil formulation when in the mouth so as to produce a desired mouth "feel".

The texture modifier employed acts to reduce the bitter taste by suspending the lipid coated granules, resulting in reduced contact in the mouth, a reduced gritty texture and more ease of swallowing.

The texture modifier is xanthan gum.

The sweetener system is a mixture of acesulfame potassium and aspartame and the texture modifier is xanthan gum.

The texture modifier is preferably present in a weight ratio of modifier: lipid coated particle between about 1:300 to about 1:3000, more preferably between about 1:500 to about 1:1500. The texture modifier comprises about 0.01 to about 5% by weight of the final granule composition, more preferably about 0.01 to about 1% by weight.

The weight ratio of texture modifier:sweetener system is between about 1:1 to about 1:1000, more preferably between about 1:10 to about 1:100.

The weight ratio of lipid coated particle:sweetener system:texture modifier in the final granule composition is between about 300:10:1 to about 3000:100:1, preferably between about 500:10:1 to about 1500:100:1.

The sweetener/texture modifier particle composition may also optionally contain other excipients such as suspension and binding agents, fillers, thickeners, flavours and bulk sweeteners.

Suitable suspension and binding agents include, but are not limited to, alkycelluloses such as methylcellulose, hydroxyalkylcelluloses such as hydroxypropylcellulose and hydroxypropylmethylcellulose, sodium carboxymethylcellulose or mixtures thereof, pregelatinised maize starch or polyvinylpyrrolidone.

Suitable fillers include sucrose, starch, lactose and microcrystalline cellulose.

Bulk sweeteners which are suitable for the purposes of the present invention include sorbitol, sucrose, or artificial sweeteners such as sodium saccharin or sodium cyclamate.

Alternatively, bulk sweeteners which are suitable for the purposes of the present invention include sorbitol, mannitol, maltitol, xylitol, fructose, glucose, sucrose, or artificial sweeteners such as sodium saccharin or sodium cyclamate.

Thickeners which are suitable for the purposes of the present invention include, but are not limited to, lecithin or aluminium stearate.

Suitable flavourings such as mint, peppermint, strawberry or tutti frutti may additionally be present in the composition.

In a preferred embodiment, the lipid coated granules of cefuroxime axetil are granulated with sucrose using an aqueous solution of polyvinylpyrrolidone (Povidone) as a binder to form the granule. A suitable flavour, such as tutti frutti flavour, is added and the composition is blended. The sweetener and texture modifier of the present invention may be blended together with the granulated particles in the form of a dry mix using conventional techniques either before, after or at the same time as addition of the flavouring agent to form the final granule composition. Alternatively, the sweetener and texture modifier may be blended with the lipid coated particles during the granulation process. During the blending process it is important to ensure that the sweetener system and texture modifier are evenly in admixture with the cefuroxime axetil lipid coated particles.

The particulate products according to the present invention may be used in pharmaceutical compositions for oral administration and may be presented as a suspension for administration, as a dry product for constitution with water or other suitable vehicle before use for administration as a suspension, or for direct administration and then washed down with water or other suitable liquid.

In a further aspect, therefore, the invention provides a pharmaceutical composition for oral administration comprising a composition according to the invention together with one or more pharmaceutical carriers or excipients.

The pharmaceutical compositions of the invention, formulated for oral administration as a suspension, may be constituted with a suitable amount of water, for use in oral administration of cefuroxime axetil. The particles will be typically presented so as to give a multidose suspension containing the equivalent of 125 mg to 5 g cefuroxime axetil or a single dose suspension containing the equivalent of 125 to 500 mg cefuroxime axetil.

Doses employed for human treatment will typically be in the range of 250 to 1000 mg cefuroxime axetil per day for adults and 80 to 500 mg per day for children, although the precise dose will depend on *inter alia* the frequency of administration.

The present invention may be further illustrated by the following examples.

### Examples

The cefuroxime axetil used in the Examples was highly pure spray dried amorphous material prepared as described in GB 2127401. The sweetener system and texture modifier were blended together with the cefuroxime axetil granules as a dry mix ensuring that they are evenly in admixture.

Examples 2-5 and 7-9 are reference examples.

### Example 1

**Cefuroxime axetil suspension 125 mg/5ml**

| Ingredients | 5mL Dose | %w/w |
|---|---|---|
| Cefuroxime axetil | 0.150 g | 3.55 |
| Stearic acid | 0.852 g | 20.19 |
| Povidone | 0.013 g | 0.31 |
| Tutti Frutti flavour | 0.100 g | 2.37 |
| Sucrose | 3.062 g | 72.56 |
| Acesulfame Potassium | 0.021 g | 0.50 |
| Aspartame | 0.021 g | 0.50 |
| Xanthan gum | 0.001 g | 0.02 |
| | | |
| Potable Water to | 5mL | |

### Example 2

**Cefuroxime axetil suspension 125 mg/5ml**

| Ingredients | 5mL Dose | %w/w |
|---|---|---|
| Cefuroxime axetil | 0.150 g | 3.55 |
| Stearic acid | 0.852 g | 20.19 |
| Povidone | 0.013 g | 0.31 |
| Tutti Frutti flavour | 0.100 g | 2.37 |
| Sucrose | 3.062 g | 72.56 |
| Sodium saccharin | 0.021 g | 0.50 |
| Aspartame | 0.021 g | 0.50 |
| Xanthan gum | 0.001 g | 0.02 |
| | | |
| Potable Water to | 5mL | |

### Example 3

### Cefuroxime axetil suspension 125 mg/5ml

| Ingredients | 5mL Dose | %w/w |
|---|---|---|
| Cefuroxime axetil | 0.150 g | 3.55 |
| Stearic acid | 0.852 g | 20.19 |
| Povidone | 0.013 g | 0.31 |
| Tutti Frutti flavour | 0.100 g | 2.37 |
| Sucrose | 3.062 g | 72.56 |
| Sodium saccharin | 0.021 g | 0.50 |
| Acesulfame Potassium | 0.021 g | 0.50 |
| Xanthan gum | 0.001 g | 0.02 |
| | | |
| Potable Water to | 5mL | |

### Example 4

### Cefuroxime axetil suspension 125 mg/5ml

| Ingredients | 5mL Dose | %w/w |
|---|---|---|
| Cefuroxime axetil | 0.150 g | 3.56 |
| Stearic acid | 0.852 g | 20.24 |
| Povidone | 0.013 g | 0.31 |
| Tutti Frutti flavour | 0.100 g | 2.38 |
| Sucrose | 3.062 g | 72.75 |
| Neohesperidin dihydrochalcone | 0.010 g | 0.24 |
| Sodium saccharin | 0.021 g | 0.50 |
| Xanthan gum | 0.001 g | 0.02 |
| | | |
| Potable Water to | 5mL | |

### Example 5

**Cefuroxime axetil suspension 125 mg/5ml**

| Ingredients | 5mL Dose | %w/w |
|---|---|---|
| Cefuroxime axetil | 0.150 g | 3.57 |
| Stearic acid | 0.852 g | 20.29 |
| Povidone | 0.013 g | 0.31 |
| Tutti Frutti flavour | 0.100 g | 2.38 |
| Sucrose | 3.062 g | 72.92 |
| Thaumatin | 0.010 mg | 2.38x10⁻⁴ |
| Sodium saccharin | 0.021 g | 0.50 |
| Xanthan gum | 0.001 g | 0.02 |
| | | |
| Potable Water to | 5mL | |

### Example 6

**Cefuroxime axetil suspension 250 mg/5ml**

| Ingredients | 5mL Dose | %w/w |
|---|---|---|
| Cefuroxime axetil | 0.300 g | 7.50 |
| Stearic acid | 1.203 g | 30.09 |
| Povidone | 0.012 g | 0.30 |
| Tutti Frutti flavour | 0.102 g | 2.55 |
| Sucrose | 2.289 g | 57.25 |
| Acesulfame Potassium | 0.045 g | 1.13 |
| Aspartame | 0.045 g | 1.13 |
| Xanthan gum | 0.002 g | 0.05 |
| | | |
| Potable Water to | 5mL | |

### Example 7

**Cefuroxime axetil suspension 250 mg/5ml**

| Ingredients | 5mL Dose | %w/w |
|---|---|---|
| Cefuroxime axetil | 0.300 g | 7.50 |
| Stearic acid | 1.203 g | 30.09 |
| Povidone | 0.012 g | 0.30 |
| Tutti Frutti flavour | 0.102 g | 2.55 |
| Sucrose | 2.289 g | 57.25 |
| Sodium saccharin | 0.045 g | 1.13 |
| Aspartame | 0.045 g | 1.13 |
| Xanthan gum | 0.002 g | 0.05 |
| | | |
| Potable Water to | 5mL | |

### Example 8

**Cefuroxime axetil suspension 250 mg/5ml**

| Ingredients | 5mL Dose | %w/w |
|---|---|---|
| Cefuroxime axetil | 0.300 g | 7.50 |
| Stearic acid | 1.203 g | 30.09 |
| Povidone | 0.012 g | 0.30 |
| Tutti Frutti flavour | 0.102 g | 2.55 |
| Sucrose | 2.289 g | 57.25 |
| Sodium saccharin | 0.045 g | 1.13 |
| Acesulfame Potassium | 0.045 g | 1.13 |
| Xanthan gum | 0.002 g | 0.05 |
| | | |
| Potable Water to | 5mL | |

### Example 9

**Cefuroxime axetil suspension 250 mg/5ml**

| Ingredients | 5mL Dose | %w/w |
|---|---|---|
| Cefuroxime axetil | 0.300 g | 7.55 |
| Stearic acid | 1.203 g | 30.28 |
| Povidone | 0.012 g | 0.30 |
| Tutti Frutti flavour | 0.102 g | 2.57 |
| Sucrose | 2.289 g | 57.62 |
| Neohesperidin dihydrochalcone | 0.020 g | 0.50 |
| Sodium saccharin | 0.045 g | 1.13 |
| | | |
| Xanthan gum | 0.002 g | 0.05 |
| | | |
| Potable Water to | 5mL | |

### Results

A taste trial was performed in which 5 volunteers assessed a suspension of the composition of Example 1 reconstituted with potable water according to the following categories:

| | |
|---|---|
| Initial taste: | sweet or bitter |
| Aftertaste: | bitter aftertaste present or absent |
| Mouthfeel: | creamy or gritty |
| Flavour: | pleasant or unpleasant |

The results of the taste trial are tabulated below:

| **Taste category** | **Volunteer response** |
|---|---|
| Initial taste | All volunteers appreciated a sweet taste in the preparation |
| Bitter aftertaste | None of the volunteers appreciated a bitter aftertaste in the preparation |
| Mouthfeel | All volunteers appreciated a creamy mouthfeel in the preparation, although some granules could be detected. |
| Flavour | All volunteers appreciated a pleasant Tutti Frutti flavour in the preparation |

Further taste trials were carried out in a number of healthy adult patients comparing a suspension of the composition of Example 1 (125mg/ml) and of Example 6 (250mg/5ml) with a suspension of compositions of cefuroxime axetil which were identical except for the absence of the sweetener system and texture modifier. Formulations in both strengths were assessed in the "fresh" form, ie freshly constituted formulations.

In a preference test design, the suspensions were compared for sweetness, bitterness, mouthfeel and overall preference. The results demonstrated in the following tables show percentages of patients preference for both a 125mg/5ml dose form and a 250mg/5ml dose form.

| **Suspension** | **Sweeter** | **Bitter** | **Better Mouthfeel** | **Preference** |
|---|---|---|---|---|
| **125mg/5ml taste trials** | | | | |
| Suspension 1^{a} | 66% | 0% | 42% | 58% |
| Suspension 2^{b} | 17% | 67% | 16% | 17% |
| Equal | 17% | 33% | 42% | 25% |
| **250mg/5ml taste trials** | | | | |
| Suspension 1^{a} | 75% | 0% | 42% | 92% |
| Suspension 2^{b} | 0% | 100% | 25% | 8% |
| Equal | 25% | 0% | 33% | 0% |

| | | | | |
|---|---|---|---|---|
| ^{a}Suspension 1 Cefuroxime axetil lipid coated particles plus a sweetener system and a texture modifier ^{b}Suspension 2 Cefuroxime axetil lipid coated particles | | | | |

The results clearly indicate that suspensions of the present invention which contain added sweeteners and texture modifier is the much preferred formula for both taste and mouthfeel.

## Claims

1. A composition comprising cefuroxime axetil in particulate form, the particles being provided with integral coatings of lipid or mixture of lipids which are insoluble in water and which disperse or dissolve on contact with gastrointestinal fluid, a bulk sweetener and a binding agent, **characterised in that** the composition further comprises in amounts sufficient to mask the bitter taste of cefuroxime axetil
- acesulfame potassium and aspartame, present in an amount together of 0.1 to 10% by weight of the final granule composition, and
- a texture modifier which is xanthan gum, present in an amount together of 0.01 to 5% by weight of the final granule composition.

2. A composition according to Claim 1 wherein the acesulfame potassium and aspartame are present in a weight ratio of 1 :10 to 10:1.

3. A composition according to claim 1 or claim 2 wherein the acesulfame potassium and aspartame are present in a weight ratio of 1:1.

4. A composition according to any one of claims 1 to 3 wherein the weight ratio of texture modifier: (acesulfame potassium and aspartame) is between 1:1 to 1:1000.

5. A composition according to any one of claims 1 to 7 wherein the weight ratio of lipid coated particulate : (acesulfame potassium and aspartame) : texture modifier is between 300:10:1 to 3000:100:1.

6. A composition according to any one of claims I to 5 wherein the bulk sweetener is sucrose.

7. A composition according to any one of claims 1 to 6 wherein the binding agent is povidone.

8. A pharmaceutical composition for oral administration comprising a composition as claimed in any one of claims 1 to 7 together with one or more pharmaceutically acceptable carriers or excipients.

9. A pharmaceutical composition according to claim 8 in the form of an aqueous suspension.

10. A pharmaceutical composition according to claim 8 in the form of granules.

## Patentansprüche

1. Zusammensetzung, umfassend Cefuroximaxetil in partikulärer Form, wobei die Partikel mit integralen Beschichtungen eines Lipids oder Gemisches von Lipiden, die in Wasser unlöslich sind und nach Berührung mit einer gastrointestinalen Flüssigkeit dispergieren oder sich auflösen, versehen sind, ein Füllsüßstoff und ein Bindemittel, **dadurch gekennzeichnet, dass** die Zusammensetzung ferner folgendes in Mengen umfasst, die ausreichen, den bitteren Geschmack von Cefuroximaxetil zu überdecken:
- Acesulfamkalium und Aspartam, vorliegend in einer Menge zusammen von 0,1 bis 10 Gew.-% der endgültigen Granalienzusammensetzung, und
- ein Textur-modifizierendes Mittel, das Xanthangummi ist, vorliegend in einer Menge zusammen von 0,01 bis 5 Gew.-% der endgültigen Granalienzusammensetzung.

2. Zusammensetzung gemäß Anspruch 1, wobei das Acesulfamkalium und Aspartam in einem Gewichtsverhältnis von 1:10 bis 10:1 vorliegen.

3. Zusammensetzung gemäß Anspruch 1 oder Anspruch 2, worin das Acesulfamkalium und Aspartam in einem Gewichtsverhältnis von 1:1 vorliegen.

4. Zusammensetzung gemäß einem der Ansprüche 1 bis 3, wobei das Gewichtsverhältnis von Textur-modifizierendem Mittel:(Acesulfamkalium und Aspartam) zwischen 1:1 und 1:1000 liegt.

5. Zusammensetzung gemäß einem der Ansprüche 1 bis 4, wobei das Gewichtsverhältnis von lipidbeschichtetem Partikel:(Acesulfamkalium und Aspartam):Textur-modifizierendem Mittel zwischen 300:10:1 bis 3000:100:1 liegt.

6. Zusammensetzung gemäß einem der Ansprüche 1 bis 5, wobei der Füllsüßstoff Saccharose ist.

7. Zusammensetzung gemäß einem der Ansprüche 1 bis 6, wobei das Bindemittel Povidon ist.

8. Pharmazeutische Zusammensetzung für die orale Verabreichung, umfassend eine Zusammensetzung gemäß einem der Ansprüche 1 bis 7 zusammen mit einem oder mehreren pharmazeutisch annehmbaren Trägern oder Exzipienten.

9. Pharmazeutische Zusammensetzung gemäß Anspruch 8 in der Form einer wässrigen Suspension.

10. Pharmazeutische Zusammensetzung gemäß Anspruch 8 in der Form von Granalien.

## Revendications

1. Composition comprenant du céfuroxime axétil sous forme particulaire, les particules étant fournies avec des enrobages intégraux de lipide ou de mélanges de lipides qui sont insolubles dans l'eau et qui se dispersent ou se dissolvent après contact avec le liquide gastro-intestinal, un édulcorant en vrac et un agent de liaison, **caractérisée en ce que** la composition comprend en outre dans des quantités suffisantes pour masquer le goût amer du céfuroxime axétil
- de l'acésulfame potassium et de l'aspartame, présents dans une quantité conjointement de 0,1 à 10 % en poids de la composition finale de granulés, et
- un modificateur de la texture qui est la gomme xanthane, présent dans une quantité conjointement de 0,01 à 5 % en poids de la composition finale de granulés.

2. Composition selon la revendication 1, dans laquelle l'acésulfame potassium et l'aspartame sont présents dans un rapport pondéral de 1/10 à 10/1.

3. Composition selon la revendication 1 ou la revendication 2, dans laquelle l'acésulfame potassium et l'aspartame sont présents dans un rapport pondéral de 1/1.

4. Composition selon l'une quelconque des revendications 1 à 3, dans laquelle le rapport pondéral du modificateur de la texture/ (acésulfame potassium et aspartame) est entre 1/1 et 1/1000.

5. Composition selon l'une quelconque des revendications 1 à 7, dans laquelle le rapport pondéral des particules enrobées de lipide/(acésulfame potassium et aspartame)/modificateur de la texture est entre 300/10/1 et 3000/100/1.

6. Composition selon l'une quelconque des revendications 1 à 5, dans laquelle l'édulcorant en vrac est le saccharose.

7. Composition selon l'une quelconque des revendications 1 à 6, dans lequel l'agent de liaison est la povidone.

8. Composition pharmaceutique pour une administration orale comprenant une composition telle que revendiquée dans l'une quelconque des revendications 1 à 7 conjointement avec un ou plusieurs supports ou excipients pharmaceutiquement acceptables.

9. Composition pharmaceutique selon la revendication 8, sous la forme d'une suspension aqueuse.

10. Composition pharmaceutique selon la revendication 8, sous la forme de granulés.
